(19) Europäisches Patentamt
European Patent Office
Office européen des brevets

(11) EP 2 362 790 B1

(12) FASCICULE DE BREVET EUROPEEN

(45) Date de publication et mention de la délivrance du brevet:
**27.05.2015 Bulletin 2015/22**

(21) Numéro de dépôt: **09752880.6**

(22) Date de dépôt: **24.09.2009**

(51) Int Cl.:
***A61M 1/34*** *(2006.01)*     ***A61M 1/14*** *(2006.01)*

(86) Numéro de dépôt international:
**PCT/FR2009/051812**

(87) Numéro de publication internationale:
**WO 2010/040927 (15.04.2010 Gazette 2010/15)**

(54) **APPAREIL DE TRAITEMENT EXTRACORPOREL DE SANG**

GERÄT ZUR EXTRAKORPORALEN BLUTBEHANDLUNG

APPARATUS FOR EXTRACORPOREAL BLOOD TREATMENT

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **06.10.2008 FR 0856758**

(43) Date de publication de la demande:
**07.09.2011 Bulletin 2011/36**

(60) Demande divisionnaire:
**15159940.4**

(73) Titulaire: **RD Nephrologie**
**34000 Montpellier (FR)**

(72) Inventeurs:
• **FICHEUX, Alain**
  **34790 Grabels (FR)**
• **ARGILES CISCART, Angel**
  **34980 Saint Gely Du Fesc (FR)**

(74) Mandataire: **Pontet Allano & Associes**
**Parc Les Algorithmes, Bâtiment Platon**
**CS 70003 Saint-Aubin**
**91192 Gif-sur-Yvette Cedex (FR)**

(56) Documents cités:
WO-A-2006/011009      DE-A1- 3 416 955
DE-C1- 19 940 624      US-A- 4 661 246

EP 2 362 790 B1

## Description

L'invention porte sur un appareil de traitement du sang à l'extérieur d'un corps.

L'invention porte plus particulièrement sur un appareil de traitement du sang à l'extérieur d'un corps en vue de l'élimination de liquide et de solutés présents dans le sang. Un tel procédé est plus communément appelé hémodialyse.

Actuellement, on connaît plusieurs types d'hémodialyses réalisées avec des appareils, appelés générateur de dialyse, comportant un filtre, appelé dialyseur, dans lequel un échange de solutés et de liquides est réalisé par le biais d'une membrane semi-perméable.

Dans les procédés dits « ultrafiltration pure », le liquide à enlever est retiré du sang par gradient de pression et les solutés à éliminer sont transportés par convection avec le liquide.

Dans d'autres procédés d'hémodialyse, un fluide d'une composition prédéterminé est introduit dans le compartiment non sanguin du dialyseur. L'élimination des liquides du sang à travers la membrane semi-perméable se fait par gradient de pression. L'échange de solutés à travers la membrane est réalisé principalement par diffusion due au gradient de concentration des solutés. Selon les pressions et la porosité de la membrane divers échanges d'eau et de solutés peuvent se produire.

En hémodiafiltration, les échanges de solutés se font par diffusion et par convection. Une quantité supplémentaire de liquide est enlevée par ultrafiltration. Un liquide de substitution est infusé dans le sang pour compenser la quantité de liquide supplémentaire enlevée.

Tous ces types d'hémodialyse permettent l'enlèvement d'un excès de liquide du sang traité. La quantité totale de liquide retiré du début à la fin du traitement (perte de poids) est l'un des paramètres les plus importants dans le traitement, et elle est généralement fixée au début du traitement comme une cible.

Un autre paramètre important est le temps total de traitement. La quantité de liquide retiré du sang traité par unité de temps est connue sous le nom de taux de perte de poids. Généralement, le taux de perte de poids est fixé à une valeur constante ou à un profil prédéfini.

En hémodiafiltration, la quantité de liquide infusé par unité de temps est le taux d'infusion. Le taux d'ultrafiltration sera déterminé comme la somme du taux de perte de poids et du taux d'infusion. La différence de pression de part et d'autre de la membrane est appelée pression transmembranaire (PTM).

Le rapport entre le taux horaire d'ultrafiltration et la pression transmembranaire est appelé coefficient d'ultrafiltration (KUF).

Les constructeurs de dispositifs de traitement donnent, pour chaque type de dialyseur, une valeur du coefficient d'ultrafiltration (KUF) mesurée in vitro avec du sang bovin standardisé. Cette valeur est généralement prise comme une constante in vivo. In vivo, pour un débit d'ultrafiltration (QINF) donné, l'adsorption dans la membrane de protéines, modifie la résistance à la convection et donc la pression trans-membranaire (PTM). Le coefficient d'ultrafiltration (KUF= QINF / PTM) n'est donc pas constant. Il varie avec les caractéristiques du sang. La composition du sang peut varier en cours de séance ou se modifier sur plusieurs séances. Si on augmente fortement le débit total ultrafiltré, comme c'est le cas en hémodiafiltration, la pression trans-membranaire augmente. Une valeur maximale de débit peut être atteinte. C'est le plateau où une augmentation de la pression trans-membranaire (PTM) n'entraîne pas d'augmentation de débit (dQuf/dPTM =0).

Cette valeur maximale du débit total ultrafiltré est généralement recherchée comme une valeur cible or la résistance convective est importante. La perte de poids entraîne une augmentation de l'hématocrite en cours de séance qui peut conduire à une hémoconcentration et la saturation de la membrane par les protéines, entraînant des alarmes de pression trans-membranaire. L'élimination des toxines, notamment de haut poids moléculaire dans ces conditions diminue. La PTM augmentant, le coefficient d'ultrafiltration (KUF) diminue.

On connaît du document DE 34 16 955 A1 un appareil de traitement de sang à l'extérieur du corps humain comprenant une enceinte de dialyse comportant une membrane semi-perméable réalisant un échange de solutés et de liquide avec le sang. L'appareil divulgué dans ce document comprend des moyens de détermination d'une valeur instantanée d'un coefficient d'ultrafiltration correspondant au rapport d'un débit d'ultrafiltration sur une différence, dite pression transmembranaire, de part et d'autre de la membrane semi-perméable, des moyens de variation du débit d'ultrafiltration, et des moyens de commande desdits moyens de variation du débit d'ultrafiltration, en fonction d'une comparaison de ladite valeur instantanée à une valeur initiale de manière à atteindre un coefficient d'ultrafiltration actuel.

Un but de l'invention est de pallier ces inconvénients.

Un autre but de l'invention est de fournir un appareil de traitement de sang à l'extérieur d'un corps présentant un meilleur rendement que les appareils actuellement connus.

L'invention permet d'atteindre les buts précités par un appareil selon la revendication 1.

L'élimination des toxines urémiques du sang par la dialyse dépend de la perméabilité hydraulique et diffusive de la membrane semi-perméable. Au cours d'une séance de traitement, pour un débit d'ultrafiltration constant, l'adsorption dans la membrane de protéines fait augmenter la résistance à la convection. La perméabilité hydraulique est modifiée. La perméabilité hydraulique est mesurée par la détermination du coefficient d'ultrafiltration (KUF) qui est égal au rapport du débit d'ultrafiltration en mL/h sur la pression transmembranaire (PTM) en mmHg.

En effet, des études menées par le(s) demandeur(s) montrent que le coefficient d'ultrafiltration varie notamment avec le débit d'ultrafiltration. Sa courbe de variation n'est pas un plateau mais une parabole. La valeur maximale du KUF (KUF max) correspond au meilleur débit de convection par rapport à la contrainte de pression. C'est la valeur de perméabilité hydraulique optimale pour la membrane. Cette valeur qui est obtenue en cours de séance tient compte des caractéristiques du traitement : composition du sang, débits, type de membrane, surface....

L'invention permet de déterminer le coefficient d'ultrafiltration optimal pour utiliser un appareil de dialyse à son état de rhéologie optimal.

Avantageusement, l'invention permet d'utiliser un appareil de dialyse à son mieux, et non pas à son maximum. En effet, le rendement d'un appareil de dialyse est optimal lorsque le coefficient d'ultrafiltration est maximal, ce qui correspond à un taux d'ultrafiltration meilleur que ceux obtenus actuellement et ce pour une moindre contrainte de pression transmembranaire. C'est une valeur qui permet d'utiliser l'appareil de dialyse à son état de rhéologie optimal.

Avantageusement, l'étape de détermination du coefficient d'ultrafiltration peut comprendre au moins une itération des étapes suivantes :

- mesure de la pression transmembranaire,
- calcul du débit d'ultrafiltration réalisée par la membrane semi-perméable, et
- calcul de la valeur de coefficient d'ultrafiltration par division dudit débit d'ultrafiltration par ladite pression transmembranaire.

Lors de l'étape de comparaison, la ou les valeurs caractéristiques préalablement déterminées peuvent comprendre :

- soit des valeurs fournies par le constructeur de l'appareil de traitement, ou
- soit des valeurs déterminées lors d'une itération précédente ou lors d'une ou plusieurs séances de traitement préalables.

Ainsi, la valeur instantanée du coefficient d'ultrafiltration peut être comparée à une ou plusieurs valeurs qui sont :

- soit fournies par le constructeur de l'appareil de traitement, ou
- soit déterminées lors d'une ou plusieurs itérations ou séances de traitement précédentes.

Selon un mode de réalisation, la valeur maximale du coefficient d'ultrafiltration peut être mesurée lors de la séance de traitement en cours par détermination de la variation dudit coefficient d'ultrafiltration en fonction du débit d'ultrafiltration, ladite détermination comprenant plusieurs itérations des étapes suivantes :

- variation du débit d'ultrafiltration
- mesure de la pression transmembranaire obtenue pour ce débit d'ultrafiltration,
- calcul da la valeur de coefficient d'ultrafiltration par division dudit débit d'ultrafiltration par ladite pression transmembranaire, et
- mémorisation dudit coefficient d'ultrafiltration calculé en association avec le débit d'ultrafiltration ;

Dans ce mode de réalisation, l'invention comprend une première itération pendant laquelle une première valeur $KUF_0$ du coefficient d'ultrafiltration est déterminée après mesure de la pression transmembranaire. Cette valeur est mise en mémoire. Puis lors d'une deuxième itération, le débit d'ultrafiltration est modifié et une nouvelle valeur $KUF_1$ du coefficient d'ultrafiltration est déterminée après mesure de la pression transmembranaire. Si $KUF_1 > KUF_0$ alors $KUF_1$ est mis en mémoire et ainsi de suite. La série d'itérations s'arrête lorsqu'on obtient $KUF_k > KUF_{k+1}$. Le débit d'ultrafiltration optimal est celui qui correspond au coefficient d'ultrafiltration $KUF_k$. Dans ce mode de réalisation, la valeur instantanée du coefficient d'ultrafiltration est comparée à une valeur d'ultrafiltration déterminée lors de l'itération précédente. Tel qu'indiqué plus haut la comparaison peut se faire par rapport à une valeur donnée par le constructeur ou déterminée lors de séances précédentes.

La détermination de la variation du coefficient d'ultrafiltration peut se faire à tout moment lors de la séance de traitement de sang. La détermination de la variation peut se faire plusieurs fois lors d'une séance de traitement pour optimiser le rendement de l'appareil de traitement. Elle peut soit se faire de manière automatique, soit être déclenchée par une intervention manuelle de la part d'un opérateur.

Tel que précisé plus haut, la variation du coefficient d'ultrafiltration en fonction du débit d'ultrafiltration se fait selon une courbe parabolique. Le débit optimal d'ultrafiltration est celui pour lequel le coefficient d'ultrafiltration est sensiblement égal à la valeur correspondant au sommet de cette courbe parabolique.

Avantageusement, les moyens de détermination de la valeur instantanée du coefficient d'ultrafiltration peuvent comprendre :

- des capteurs de mesure de la pression transmembranaire, et plus particulièrement plusieurs capteurs disposés au niveau de chaque entrée et sortie de l'enceinte de dialyse et mesurant la pression au niveau de chaque entrée et sortie de l'enceinte de dialyse ;
- des moyens de détermination du débit d'ultrafiltration, et
- des moyens de calcul d'une valeur du coefficient d'ultrafiltration par division dudit débit d'ultrafiltration par ladite pression transmembranaire.

Chacun des capteurs et les moyens de calculs peuvent être reliés au module de commande.

L'appareil selon l'invention peut avantageusement comprendre des moyens de mémorisation agencés pour mémoriser au moins une valeur de coefficient d'ultrafiltration pour une valeur de débit d'ultrafiltration.

Par ailleurs, les moyens de commande peuvent comprendre des moyens d'exécution d'instructions mémorisées dans des moyens de mémorisation, éventuellement intégrés dans le module de commande, lesdites instructions réalisant un calcul de la valeur maximale du coefficient d'ultrafiltration lors d'une séance de traitement en cours par détermination de la variation dudit coefficient d'ultrafiltration en fonction du débit d'ultrafiltration, ladite détermination comprenant plusieurs itérations des étapes suivantes :

- variation du débit d'ultrafiltration
- mesure de la pression transmembranaire obtenue pour ce débit d'ultrafiltration,
- calcul de la valeur de coefficient d'ultrafiltration par division dudit débit d'ultrafiltration par ladite pression transmembranaire, et
- mémorisation, dans les moyens de mémorisation, dudit coefficient d'ultrafiltration calculé en association avec le débit d'ultrafiltration ;

Avantageusement, des moyens de déclenchements manuels peuvent être agencés pour un déclenchement manuel :

- de la détermination de la valeur instantanée du coefficient d'ultrafiltration, et/ou
- de la détermination de la valeur maximale du coefficient d'ultrafiltration lors d'une séance de traitement en cours.

Ainsi, l'opérateur peut à tout moment et aussi souvent que nécessaire déclencher la détermination de la valeur instantanée du coefficient d'ultrafiltration. Si la valeur d'ultrafiltration est inférieure, d'une valeur prédéterminée, à la valeur maximale de ce coefficient alors le module de commande peut effectuer un réglage du débit d'ultrafiltration pour atteindre la valeur maximale du coefficient d'ultrafiltration.

L'appareil selon l'invention peut être mis en oeuvre pour :

- un traitement de sang à l'extérieur du corps humain par ultrafiltration pure,
- un traitement de sang à l'extérieur du corps humain par hémodialyse, ou
- un traitement de sang à l'extérieur du corps humain par hémodiafiltration.

Avantageusement, l'enceinte de dialyse est jetable.

D'autres avantages et caractéristiques de l'invention apparaîtront à l'examen de la description détaillée d'un mode de mise en oeuvre nullement limitatif, et des dessins annexés sur lesquels :

- la figure 1 illustre des exemples de profil de variation du coefficient d'ultrafiltration en fonction du débit d'ultrafiltration pour un même type de dialyseur,
- la figure 2 est une représentation schématique d'un appareil pouvant être mis en oeuvre pour un traitement d'ultrafiltration pure, d'hémodialyse ou d'hémodiafiltration, et
- la figure 3 est une représentation schématique d'un exemple d'un appareil selon l'invention.

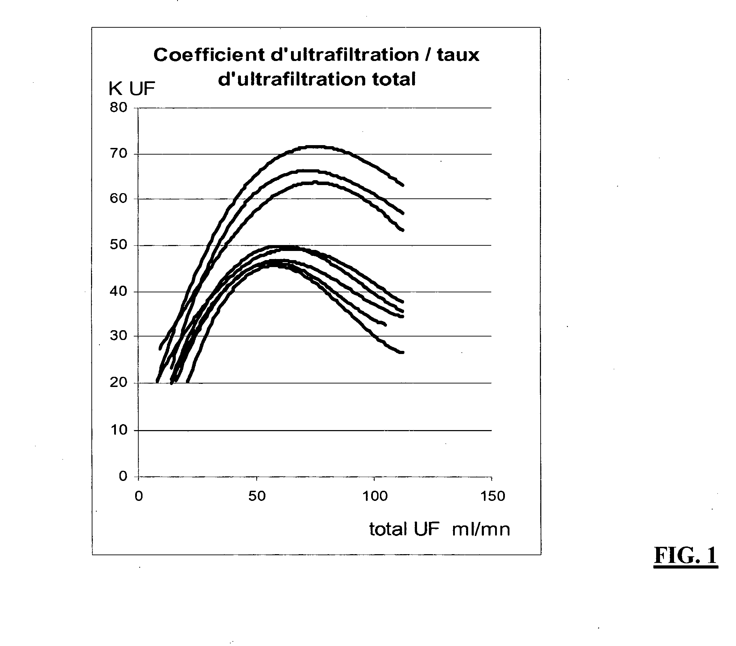

La figure 1 montre plusieurs exemples de variations du coefficient d'ultrafiltration en fonction du débit d'ultrafiltration pour un même type de dialyseur. Tel que représenté en figure 1, des mesures montrent que la courbe de variation du coefficient d'ultrafiltration KUF en fonction du débit d'ultrafiltration, croît, passe par un maximum puis décroît. La forme générale de la courbe est une forme de parabole quel que soit le patient ou le moment de la séance. En revanche les valeurs de KUF ainsi que les valeurs maximales sont différentes.

Le coefficient d'ultrafiltration n'est donc pas une constante. Il varie selon de multiples facteurs dont les caractéristiques de la membrane, la surface, la composition du sang ou les débits. C'est un nouveau paramètre de traitement.

La surveillance du coefficient d'ultrafiltration pendant le traitement permet de contrôler les conditions de travail de l'appareil d'ultrafiltration et de suivre, au cours du traitement, les deux variables dans le filtre, la performance et les variations des caractéristiques du sang du patient pour améliorer le rendement de la membrane semi-perméable utilisée pour épurer le sang du patient dans une situation donnée.

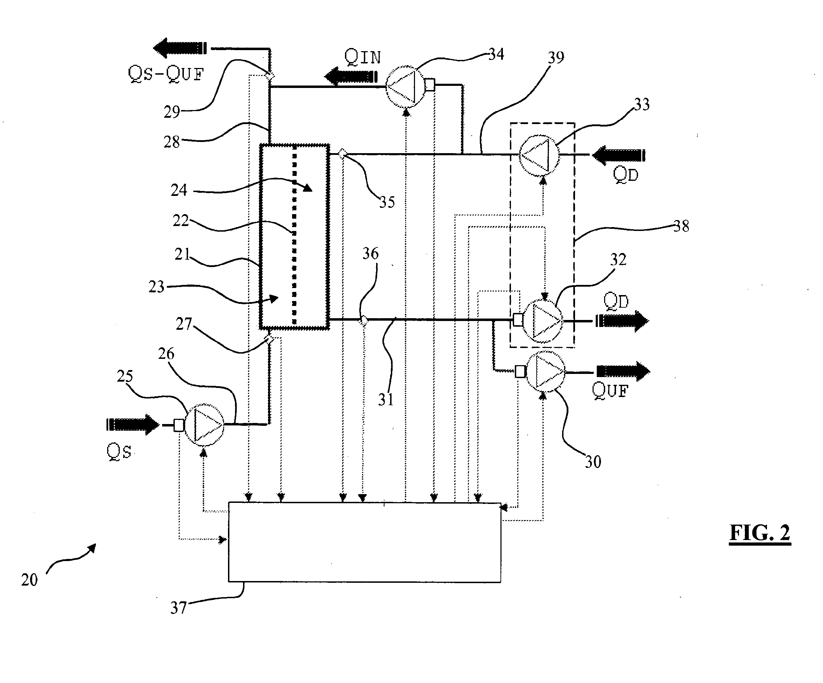

Nous allons maintenant décrire, en référence à la figure 2, un appareil 20 de traitement extracorporel de sang à l'extérieur du corps selon l'invention. L'appareil 20 comprend une enceinte de traitement 21 comportant une membrane semi-perméable 22 qui sépare le volume interne de l'enceinte de traitement 21 en deux compartiments : le compartiment 23 qui reçoit le sang à traiter et le compartiment 24 qui reçoit par exemple le dialysat. L'appareil 20 est approprié pour l'exécution de l'ultrafiltration pure, de l'hémodialyse ou de l'hémodiafiltration.

**Cas d'un traitement par ultrafiltration pure**

L'appareil de traitement 20 comprend en outre une pompe 25, par exemple de type péristaltique, agencé pour faire circuler le sang du patient par la ligne 26 vers l'enceinte de traitement 21, aussi appelé dialyseur 21, à un débit régulé et contrôlé égal à $Q_S$.

La pression du sang est mesurée par un capteur 27 avant le compartiment 23 du dialyseur 21. Le sang est en contact avec la membrane semi-perméable 22.

Le retour du sang traité vers le patient se fait par la ligne 28. La pression est mesurée par un capteur 29.

En ultrafiltration pure, une pompe 30 située sur une ligne 31, connectée au compartiment 24 du dialyseur 21 fonctionne à un débit contrôlé précisément par un dispositif de type connu et adapté pour mesurer un débit d'ultrafiltration égal au taux de perte de poids $Q_{UF}$.

Des pompes 32, 33 et 34 sont occlusives et à l'arrêt. Un capteur 35 mesure la pression dans cette ligne 31 avant le dialyseur 21.

Le liquide du sang est ultrafiltré au travers de la membrane semi-perméable 22 vers le compartiment 24 du dialyseur et vers la pompe 30. Un capteur 36 mesure la pression.

Le débit en retour du dialyseur 21 vers le patient se fait à un débit égal à $Q_S$-$Q_{UF}$, $Q_{UF}$ étant le taux de perte de poids.

Un contrôleur 37 reçoit des données de pression des capteurs 27, 29, 35 et 36 et des données de débits des pompes 25, 30, 32, 33, 34. Le contrôleur 37 est agencé de manière à contrôler le débit des pompes 25, 30, 32, 33, 34.

Ce contrôleur 37 calcule la pression transmembranaire PTM sur la base des valeurs des pressions mesurées aux 4 points par les capteurs 27, 29, 35 et 36. La PTM est égale à la moyenne des pressions du compartiment de sang 23 moins la moyenne des pressions du compartiment dialysat 24. En l'absence de quatre capteurs, la PTM pourra également être déterminée mais de manière moins précise par deux capteurs, l'un situé sur le retour du sang, ligne 28, et l'autre sur le retour du dialysat, ligne 31 ou par trois capteurs avec le troisième capteur positionné sur la ligne de sang à l'entrée du dialyseur, c'est-à-dire la ligne 26.

Pour la première mesure du coefficient d'ultrafiltration, le contrôleur 37 arrête la pompe d'ultrafiltration 30 et attend la stabilisation des mesures de pression, environ 1 min puis calcule la PTM et met cette valeur, égale à $PTM_0$ en mémoire dans des moyens de mémorisation intégrés au contrôleur 37. Il augmente ensuite la pompe 30 à la valeur de débit égale à une perte de poids programmée, il attend la stabilisation des mesures, environ 1 min, puis calcule la pression transmembranaire $PTM_i$. La valeur du coefficient d'ultrafiltration calculée sera égale au taux de perte de poids $Q_{UF}$ divisé par la valeur ($PTM_i$-$PTM_0$).

Régulièrement, en cours de séance, le contrôleur 37 procédera à un ajustement de la valeur du coefficient d'ultrafiltration à la pompe 30 en arrêtant la pompe d'ultrafiltration 30 pour un temps de stabilisation permettant d'actualiser la valeur $PTM_0$.

Le coefficient d'ultrafiltration sera affiché par l'appareil 20 sur des moyens de visualisation (non représentés) et pourra être comparé avec des valeurs mises en mémoire, par exemple, des valeurs caractéristiques pour le type de dialyseur ou pour le patient ou des valeurs de début de séance ou de valeurs mesurées lors de séances précédentes de ce patient, ceci afin de contrôler les variations inter séances ou intra séance et induire une procédure d'amélioration du rendement de l'appareil 20 ou prévoir des messages d'alerte ou des alarmes via le contrôleur 37.

Par appui sur un bouton de mise en marche du control, le contrôleur 37 arrête la pompe d'ultrafiltration 30 et attend la stabilisation des mesures de pression, environ 1 min, puis calcule la PTM et met cette valeur, égale à $PTM_0$ en mémoire. Le contrôleur 37 augmente ensuite le débit de la pompe d'ultrafiltration 30, par paliers, jusqu'à la valeur $Q_{UF_x}$ et pour un temps de stabilisation prédéterminé. A la fin de chaque palier, la PTM sera égale à $PTM_x$. Puis, le coefficient d'ultrafiltration est déterminé avec la formule :

$$\frac{Q_{UFx}}{PTM_x - PTM_0}$$

Pour le premier palier la valeur du coefficient d'ultrafiltration sera comparée avec des valeurs caractéristiques de différents types de membranes mises en mémoire et avec la perte de poids programmée du patient.

Selon le résultat, apparenté au type de membrane, différents paliers seront prévus par le calculateur 37 à la condition que la perte de poids restante soit supérieure à la somme des pertes de poids engendrées pour tous les paliers prévus. Le débit d'ultrafiltration et la PTM devront être inférieurs à des limites programmées sinon la valeur optimale du coefficient d'ultrafiltration sera considérée comme celle obtenue à la première limite.

La valeur du coefficient d'ultrafiltration déterminée à la fin de chaque palier sera mise en mémoire et comparée avec les précédentes valeurs. Si cette dernière valeur est inférieure aux valeurs précédentes d'une certaine valeur préprogrammée alors il n'y aura pas de palier supplémentaire. Le calculateur 37 calculera une courbe de tendance des valeurs et ajustera le débit de la pompe d'ultrafiltration 30 pour obtenir le coefficient d'épuration maximal.

Le calculateur 37 engendrera un signal indiquant que la valeur optimale est atteinte.

Une confirmation de la valeur de débit de la pompe d'ultrafiltration 30 sera demandée par le calculateur 37. Si la valeur est confirmée, la pompe d'ultrafiltration 30 sera maintenue à cette valeur pendant le temps prévu ou arrêtée lorsque la perte de poids prévue sera atteinte. Le coefficient d'ultrafiltration sera calculé en permanence. Régulièrement, en cours de séance, le calculateur 37 procédera à un ajustement de la valeur en arrêtant la pompe d'ultrafiltration 30 pour un temps de stabilisation permettant d'actualiser la valeur $PTM_0$.

Si la valeur n'est pas confirmée, la pompe d'ultrafiltration 30 sera maintenue à la valeur programmée de perte de poids par rapport au temps de dialyse.

L'appareil de traitement 20 permet également l'exécution d'un traitement par hémodialyse.

## Cas du traitement par hémodialyse

Le circuit du sang est inchangé par rapport à l'ultrafiltration pure.

Les pompes 32 et 33 permettent la circulation du dialysat dans le dialyseur 21 et plus précisément dans le compartiment 24 du dialyseur 21. Le dialysat passe par la membrane semi-perméable 22 vers le compartiment 23 du dialyseur 21. Le débit est égal à $Q_D$. Le retour du dialyseur 21 par la ligne 31 se fait à un débit égal $Q_D$ augmenté du taux de perte de poids.

Les capteurs de pression 35 et 36 permettent le calcul de la PTM. La circulation du dialysat est en général contrôlée par un module d'équilibre volumétrique 38 dont la particularité est que le débit $Q_D$ qui sort de ce module 38 est identique à celui qui en revient. La perte de poids est réalisée par la pompe 30. Le débit $Q_{UF}$ est égal au taux de perte de poids.

A la place du module d'équilibre volumétrique 38 la circulation du dialysat peut également être réalisée par deux pompes, l'une à l'entrée du dialyseur et l'autre à la sortie. La pompe de sortie ayant un débit $Q_D$ égal à celle de la pompe d'entrée augmenté du taux de perte de poids $Q_{UF}$. Un dispositif de type connu mesure et contrôle précisément les débits.

La mesure du coefficient d'ultrafiltration et son ajustement sont réalisés de manière comparable à celle décrite pour l'ultrafiltration pure.

L'appareil de traitement 20 peut aussi être utilisé pour un traitement de sang par hémodiafiltration.

## Cas du traitement par hémodiafiltration

Un liquide est infusé chez le patient en continu par la pompe 34 à un débit contrôlé par un dispositif connu (non représenté) tel que des moyens de pesée ou de contrôle par ultrasons. Ce liquide peut être prélevé dans des poches stériles, ou, sous certaines conditions d'asepsie et de qualité de liquide, dans le circuit dialysat sur la ligne 39. Cette dernière technique est nommée hémodiafiltration en ligne.

L'appareil est en hémodialyse, le dialysat circule dans le dialyseur 21. Une partie du dialysat est prélevé par la pompe 34 à un débit $Q_{IN}$. Le débit dans la ligne 39 à l'entrée du dialyseur 21 est donc égal à $QD-Q_{IN}$. Le débit de sortie du dialyseur 21, à la ligne 31, est égal donc égal à $Q_D+Q_{UF}$, puisque la machine fonctionne sur le principe de l'hémodialyse. Une quantité de liquide à un débit égal à $Q_{IN}$ est donc ultrafiltrée du sang pour maintenir le débit égal à $Q_D+Q_{UF}$ en sortie de dialyseur 21. Au niveau du compartiment sanguin, le débit de sang à la sortie du dialyseur 21 est égal à $Q_S-Q_{UF}-Q_{IN}$. La pompe 34 infusant à un débit égal à $Q_{IN}$, le débit de retour du sang vers le patient est donc égal à $Q_S-Q_{UF}$, soit le débit identique à l'hémodialyse.

Le liquide d'infusion peut être injecté à la sortie de la ligne à sang du dialyseur, la ligne 28 (post dilution), ou à l'entrée, la ligne 26 (pré dilution).

Cette technique de dialyse permet d'augmenter l'ultrafiltration à l'intérieur du dialyseur 21. Le taux d'ultrafiltration de la membrane 22 est alors égal à $Q_{IN}+Q_{UF}$. Le coefficient d'ultrafiltration (KUF) est le rapport entre le taux horaire d'ultrafiltration, donc dans ce cas : $Q_{IN}+Q_{UF}$, et la pression trans-membranaire

Pour la première mesure du coefficient d'ultrafiltration, le contrôleur 37 arrête la pompe d'ultrafiltration 30 et la pompe d'infusion 34. Il attend la stabilisation des mesures de pression, environ 1 minute, puis calcule la PTM et met cette valeur, égale à $PTM_0$ en mémoire. Il remet en fonction la pompe d'ultrafiltration 30 à la valeur précédente puis augmente ensuite la pompe d'infusion 34 à la valeur prévue par l'utilisateur et programmée en début de séance, il attend la stabilisation des mesures, environ 1 minute, puis calcule la pression transmembranaire $PTM_i$. La valeur du coefficient d'ultrafiltration calculée sera égale au taux d'ultrafiltration $Q_{INi}+Q_{UF}$ divisé par la valeur ($PTM_i-PTM_0$).

Régulièrement, en cours de séance, le contrôleur 37 procédera à un ajustement de la valeur en arrêtant les pompes d'ultrafiltration 30 et d'infusion 34 pour un temps de stabilisation permettant d'actualiser la valeur $PTM_0$.

Le coefficient d'ultrafiltration sera affiché par la machine sur des moyens de visualisation connectés au contrôleur 37 et pourra être comparé avec des valeurs mises en mémoire, par exemple, des valeurs caractéristiques pour le type de dialyseur ou pour le patient ou des valeurs de début de séance ou des valeurs obtenues lors de séances précédentes pour ce patient, ceci afin de contrôler les variations inter séances ou intra séance et induire une amélioration du fonctionnement de l'appareil 20 ou des messages d'alerte ou des alarmes via le contrôleur 37.

Les conditions de perméabilité optimale seront recherchées, dans ce cas, en faisant varier le débit de la pompe d'infusion 34.

Par appui sur un bouton de mise en marche du control, le contrôleur 37 arrête les pompes d'infusion 34 et d'ultrafiltration 30 et attend la stabilisation des mesures de pression, environ 1 minute, puis calcule la PTM et met cette valeur, égale à $PTM_0$ en mémoire. Il va ensuite remettre la pompe d'ultrafiltration 30 en marche à la valeur programmée puis va augmenter, par paliers d'une valeur et d'un temps de stabilisation prédéterminé, la pompe d'infusion 34. A la fin de chaque palier il va déterminer le coefficient d'ultrafiltration avec la formule : ($Q_{INx}+Q_{UF}$) divisé par la valeur ($PTM_x-PTM_0$). Le débit d'ultrafiltration et la PTM devront être inférieurs à des limites programmées sinon la valeur optimale du coefficient d'ultrafiltration sera considérée comme celle obtenue à la première limite. A la fin de chaque palier la valeur du coefficient d'ultrafiltration sera mise en mémoire par le contrôleur 37 et comparée avec les précédentes valeurs mises en mémoire. Si cette dernière valeur est inférieure aux précédentes d'une certaine valeur programmée alors il n'y aura pas de palier supplémentaire. Le contrôleur calculera une courbe de tendance des valeurs telles que représentées en figure 1 et ajustera le débit de la pompe d'infusion 34 pour obtenir le coefficient d'épuration maximum.

Le contrôleur 37 engendrera un signal indiquant que la valeur optimale est atteinte

Une confirmation de la valeur de débit de la pompe d'ultrafiltration 30 sera demandée par le contrôleur 37. Si la valeur est confirmée, la pompe d'infusion 34 sera maintenue à cette valeur. Le coefficient d'ultrafiltration sera calculé en permanence. Régulièrement, en cours de séance, le calculateur 37 procédera à un ajustement de la valeur en arrêtant la pompe d'ultrafiltration 30 pour un temps de stabilisation permettant d'actualiser la valeur $PTM_0$. En cours de séance, des recherches de la valeur optimale pourront être effectuées soit manuellement, soit par programmation soit en raison d'une variation du KUF mesuré dans des limites prédéfinies. Ceci permettra, par exemple, d'éviter la coagulation du circuit sanguin.

Si la valeur de débit de la pompe d'ultrafiltration 30 n'est pas confirmée, la pompe d'infusion 34 sera maintenue à la valeur programmée.

Les caractéristiques de la courbe du KUF /UF (forme et maximum) permettront de vérifier si le dialyseur 21 est adapté au patient.

Cette courbe, est une caractéristique d'un patient, non seulement pour un seul traitement, mais aussi au cours de sa maladie. Certaines modifications de la composition du sang pourront être détectées en comparant les profils de courbe dans une analyse historique, par exemple dernière ou précédentes séances, afin de réduire les complications par un éventuel traitement préventif.

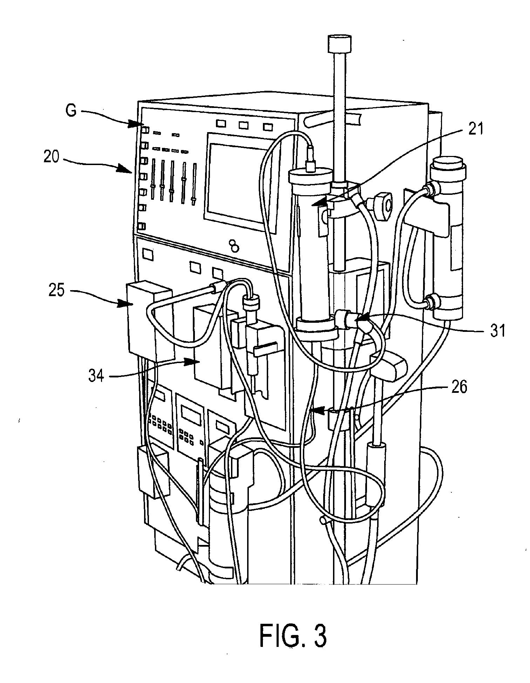

La figure 3 est une représentation schématique d'un exemple d'un appareil 20 selon l'invention. On aperçoit sur cette figure le dialyseur 21 comprenant la membrane semi perméable, la pompe 25 permettant de faire circuler le sang dans la ligne 26 ainsi que la pompe infusion 34 et la ligne 31. Par ailleurs, l'appareil 20 selon l'invention comprend en outre un générateur G pour alimenter en énergie les différents composants de l'appareil 20.

Le dialyseur 21 se trouve à l'extérieur de l'appareil 20 selon l'invention et peut être changé facilement en déconnectant des lignes 26, 28, 31 et 39.

L'invention permet d'améliorer le fonctionnement d'un appareil de traitement extracorporel du sang en fonction des conditions relatives au patient au début du traitement.

Un autre avantage de l'invention est qu'elle est adaptable à la situation du patient et aux conditions d'épuration qui varient au cours d'un traitement.

Par ailleurs, l'appareil selon l'invention comprend un contrôleur qui peut détecter les changements de paramètres en fonction de la composition du sang et peut donc fournir une analyse historique utile pour améliorer l'épuration.

L'appareil selon l'invention permet de détecter une hausse anormale de l'hématocrite et donc de prévenir la coagulation de la membrane semi-perméable.

Bien sûr, l'invention n'est pas limitée aux exemples qui viennent d'être décrits et de nombreux aménagements peuvent être apportés à ces exemples sans sortir du cadre de l'invention.

## Revendications

1. Appareil (20) de traitement de sang à l'extérieur du corps humain comprenant une enceinte de dialyse (21) comportant une membrane semi-perméable (22) réalisant un échange de solutés, dit dialyse, et de liquide, dit ultrafiltration, avec le sang, ledit appareil (20) comprenant :

   - des moyens de détermination d'une valeur, dite instantanée, d'un coefficient d'ultrafiltration correspondant au rapport d'un débit d'ultrafiltration sur une différence de pression, dite pression transmembranaire, de part et d'autre de ladite membrane semi-perméable, et
   - des moyens (30) de variation du débit d'ultrafiltration ;

   **caractérisé en ce qu'**il comprend en outre des moyens (37) de commande desdits moyens (30) de variation du débit d'ultrafiltration, en fonction d'une comparaison de ladite valeur instantanée à une valeur dite maximale, de manière à atteindre un coefficient d'ultrafiltration maximale.

2. Appareil (20) selon la revendication 1, **caractérisé en ce que** les moyens de détermination de la valeur instantanée du coefficient d'ultrafiltration comprennent :

   - des moyens (27,29,35,36,37) de mesure de la pression transmembranaire,
   - des moyens (25,30,32,33,34) de mesure du débit d'ultrafiltration réalisée par la membrane semi-perméable, et
   - des moyens (37) de calcul de la valeur de coefficient d'ultrafiltration à partir dudit débit d'ultrafiltration et de ladite pression transmembranaire.

3. Appareil (20) selon l'une quelconque des revendications 1 ou 2, **caractérisé en ce qu'**il comprend en outre des moyens pour mémoriser au moins une valeur de coefficient d'ultrafiltration pour une valeur de débit d'ultrafiltration.

4. Appareil selon l'une quelconque des revendications 1 à 3, **caractérisé en ce que** les moyens de commande (37) comprennent des moyens d'exécution d'instructions mémorisées dans des moyens de mémorisation, lesdites instructions réalisant un calcul de la valeur maximale du coefficient d'ultrafiltration lors d'une séance de traitement en cours par détermination de la variation dudit coefficient d'ultrafiltration en fonction du débit d'ultrafiltration, ladite détermination comprenant au moins une itération des étapes suivantes :

- variation du débit d'ultrafiltration
- mesure de la pression transmembranaire obtenue pour ce débit d'ultrafiltration,
- calcul de la valeur du coefficient d'ultrafiltration par division dudit débit d'ultrafiltration par ladite pression transmembranaire, et
- mémorisation dudit coefficient d'ultrafiltration calculé en association avec le débit d'ultrafiltration.

5. Appareil (20) selon l'une quelconque des revendications 1 à 4, **caractérisé en ce qu'**il comprend en outre des moyens de déclenchement manuel :

- de la détermination de la valeur instantanée du coefficient d'ultrafiltration, et/ou
- de la détermination de la valeur maximale du coefficient d'ultrafiltration lors d'une séance de traitement en cours.

6. Appareil (20) selon l'une quelconque des revendications 1 à 5, **caractérisé en ce que** l'enceinte de dialyse est jetable.

**Patentansprüche**

1. Gerät (20) für die extrakorporale Blutbehandlung, außerhalb des menschlichen Körpers, enthaltend einen Dialysebehälter (21) mit einer semipermeablen Membran (22), über die ein Austausch von gelösten Stoffen, die sogenannte Dialyse, und von Flüssigkeit, die sogenannte Ultrafiltration, mit Blut erfolgt, wobei das Gerät (20) enthält:

- Einrichtungen zum Bestimmen eines sogenannten Momentanwerts eines Ultrafiltrationskoeffizienten, der dem Verhältnis einer Ultrafiltrationsrate zu einer Druckdifferenz, dem sogenannten Transmembrandruck, beiderseits der semipermeablen Membran entspricht, und
- Einrichtungen (30) zum Ändern der Ultrafiltrationsrate,

**dadurch gekennzeichnet, dass** es ferner eine Steuereinrichtung (37) zum Steuern der Änderungseinrichtung (30) zum Ändern der Ultrafiltrationsrate in Abhängigkeit von einem Vergleich des Momentanwerts mit einem sogenannten Höchstwert umfasst, so dass ein maximaler Ultrafiltrationskoeffizient erreicht wird.

2. Gerät (20) nach Anspruch 1, **dadurch gekennzeichnet, dass** die Einrichtung zum Bestimmen des Momentanwerts des Ultrafiltrationskoeffizienten enthält:

- eine Einrichtung (27, 29, 35, 36, 37) zum Messen des Transmembrandrucks,
- eine Einrichtung (25, 30, 32, 33, 34) zum Messen der Ultrafiltrationsrate, die über die semipermeable Membran erfolgt, und
- eine Einrichtung (37) zum Berechnen des Wertes des Ultrafiltrationskoeffizienten ausgehend von der Ultrafiltrationsrate und des Transmembrandrucks.

3. Gerät (20) nach einem der Ansprüche 1 oder 2, **dadurch gekennzeichnet, dass** es ferner eine Einrichtung aufweist, um zumindest einen Wert des Ultrafiltrationskoeffizienten bei einem Wert der Ultrafiltrationsrate abzuspeichern.

4. Gerät nach einem der Ansprüche 1 bis 3, **dadurch gekennzeichnet, dass** die Steuereinrichtung (37) eine Einrichtung zum Durchführen von in einer Speichereinrichtung abgespeicherten Anweisungen enthält, wobei die Anweisungen eine Berechnung des Höchstwertes des Ultrafiltrationskoeffizienten bei einer laufenden Behandlungssitzung durch Bestimmung der Änderung des Ultrafiltrationskoeffizienten in Abhängigkeit von der Ultrafiltrationsrate durchführen, wobei die Bestimmung zumindest eine Wiederholung der nachfolgenden Schritte umfasst:

- Ändern der Ultrafiltrationsrate,
- Messen des bei dieser Ultrafiltrationsrate erhaltenen Transmembrandrucks,
- Berechnen des Werts des Ultrafiltrationskoeffizienten durch Division der Ultrafiltrationsrate durch den Transmembrandruck, und
- Abspeichern des errechneten Ultrafiltrationskoeffizienten in Verbindung mit der Ultrafiltrationsrate.

5. Gerät (20) nach einem der Ansprüche 1 bis 4, **dadurch gekennzeichnet, dass** es ferner eine Einrichtung zum manuellen Auslösen von

- der Bestimmung des Momentanwerts des Ultrafiltrationskoeffizienten und/oder
- der Bestimmung des Höchstwerts des Ultrafiltrationskoeffizienten bei einer laufenden Behandlungssitzung

enthält.

**6.** Gerät (20) nach einem der Ansprüche 1 bis 5, **dadurch gekennzeichnet, dass** der Dialysebehälter ein Einwegbehälter ist.

**Claims**

**1.** Apparatus (20) for treating blood outside the human body comprising a dialysis chamber (21) comprising a semi-permeable membrane (22) carrying out an exchange of solutes, referred to as dialysis, and of liquid, referred to as ultrafiltration, with the blood, said apparatus (20) comprising:

- means of determination of a value, said instantaneous value, of an ultrafiltration coefficient corresponding to the ratio of an ultrafiltration flow rate to a difference in pressure, said transmembrane pressure, on either side of said semi-permeable membrane,
- means (30) of variation of the ultrafiltration flow rate;

**characterised in that** it also comprises means (37) for controlling said means (30) of variation of the ultrafiltration flow rate, as a function of a comparison of said instantaneous value to a value, said maximum value, so as to reach a maximum ultrafiltration coefficient.

**2.** Apparatus (20) according to claim 1, **characterized in that** the means of determination of the instantaneous ultrafiltration coefficient value include:

- means (27,29,35,36,37) of measurement of the transmembrane pressure,
- means (25,30,32,33,34) of measurement of the ultrafiltration flow rate achieved by the semi-permeable membrane, and
- means (37) of calculation of the ultrafiltration coefficient value from said ultrafiltration flow rate and said transmembrane pressure.

**3.** Apparatus (20) according to any one of claims 1 or 2, **characterized in that** it also comprises means for storing at least one ultrafiltration coefficient value for an ultrafiltration flow rate value.

**4.** Apparatus according to any one of claims 1 to 3, **characterized in that** the means for controlling (37) comprise means of execution of instructions stored in storage means, said instructions carrying out a calculation of the maximum ultrafiltration coefficient value during a treatment session in progress by determination of the variation of said ultrafiltration coefficient as a function of the ultrafiltration flow rate, said determination comprising at least one iteration of the following steps:

- variation of the ultrafiltration flow rate,
- measurement of the transmembrane pressure obtained for this ultrafiltration flow rate,
- calculation of the ultrafiltration coefficient value by dividing said ultrafiltration flow rate by said transmembrane pressure, and
- storage of said ultrafiltration coefficient calculated in association with the ultrafiltration flow rate.

**5.** Apparatus (20) according to any one of claims 1 to 4, **characterized in that** it also comprises means for manual activation:

- of the determination of the instantaneous ultrafiltration coefficient value, and/or
- of the determination of the maximum ultrafiltration coefficient value during a treatment session in progress.

**6.** Apparatus (20) according to any one of claims 1 to 5, **characterized in that** the dialysis chamber is disposable.

**Coefficient d'ultrafiltration / taux d'ultrafiltration total**

K UF

total UF ml/mn

**FIG. 1**

**FIG.2**

FIG. 3

**EP 2 362 790 B1**

**RÉFÉRENCES CITÉES DANS LA DESCRIPTION**

**Documents brevets cités dans la description**

- DE 3416955 A1